# EUROPÄISCHE PATENTSCHRIFT

(11) **EP 2 997 957 B1**
(45) Veröffentlichungstag und Bekanntmachung des Hinweises auf die Patenterteilung: **26.08.2020**
(21) Anmeldenummer: 15185551.7
(22) Anmeldetag: 16.09.2015
(51) Int. Cl.: A61K 8/34, A61K 8/37, A61K 8/55, A61P 1/02, A61K 31/20

(54) **NEUARTIGE ZUSAMMENSETZUNG FÜR DIE MUNDPFLEGE**
NOVEL COMPOSITION FOR ORAL CARE
NOUVELLE COMPOSITION POUR L'HYGIENE BUCCALE

(30) Priorität: 17.09.2014 DE 102014113447
(43) Veröffentlichungstag der Anmeldung: 23.03.2016
(73) Patentinhaber: theranovis GmbH & Co. KG, 55411 Bingen (DE)
(72) Erfinder: PATEL, Ashokkumar, Minnetrista, MN Minnesota 55344 (US)
(74) Vertreter: Beyer, Carsten

(56) Entgegenhaltungen:
- WO-A1-00/61113
- WO-A2-2009/092040
- Anonymous: "isopropyl myristate mouthwash | Mistral How To", , 5. März 2012 (2012-03-05), XP055239728, Gefunden im Internet: URL:https://mistralhowto.wordpress.com/tag /isopropyl-myristate-mouthwash/ [gefunden am 2016-01-08]
- Anonymous: "Dentyl pH - Wikipedia, the free encyclopedia", , 15. Juli 2012 (2012-07-15), XP055239721, Gefunden im Internet: URL:https://web.archive.org/web/2012071505 4306/http://en.wikipedia.org/wiki/Dentyl_p H [gefunden am 2016-01-08]
- S. SHAPIRO: "The inhibitory action of fatty acids on oral bacteria", ORAL MICROBIOLOGY AND IMMUNOLOGY., Bd. 11, Nr. 5, 1. Oktober 1996 (1996-10-01), Seiten 350-355, XP055239452, DK ISSN: 0902-0055, DOI: 10.1111/j.1399-302X.1996.tb00193.x
- PARSAEE SIAMAK ET AL: "In-vitro release of diclofenac diethylammonium from lipid-based formulations", INTERNATIONAL JOURNAL OF PHARMACEUTICS, ELSEVIER BV, NL, Bd. 241, Nr. 1, 8. Juli 2002 (2002-07-08), Seiten 185-190, XP002523685, ISSN: 0378-5173, DOI: 10.1016/S0378-5173(02)00238-7
- ABDEL-BAR HEND MOHAMED ET AL: "Evaluation of brain targeting and mucosal integrity of nasally administrated nanostructured carriers of a CNS active drug, clonazepam.", JOURNAL OF PHARMACY & PHARMACEUTICAL SCIENCES : A PUBLICATION OF THE CANADIAN SOCIETY FOR PHARMACEUTICAL SCIENCES, SOCIÉTÉ CANADIENNE DES SCIENCES PHARMACEUTIQUES 2013, Bd. 16, Nr. 3, 2013, Seiten 456-469, XP002752780, ISSN: 1482-1826
- KINNUNEN T ET AL: "ANTIBACTERIAL AND ANTIFUNGAL PROPERTIES OF PROPYLENE GLYCOL, HEXYLENE GLYCOL, AND 1,3-BUTYLENE GLYCOL IN VITRO", ACTA DERMATO-VENEREOLOGICA, TAYLOR & FRANCIS LTD, UNITED KINGDOM, vol. 71, no. 2, 1 January 1991 (1991-01-01), pages 148-150, XP000670892, ISSN: 0001-5555
- T. Takushige ET AL: "Endodontic treatment of primary teeth using a combination of antibacterial drugs", INTERNATIONAL ENDODONTIC JOURNAL, vol. 37, no. 2, 1 February 2004 (2004-02-01), pages 132-138, XP055352333, BL ISSN: 0143-2885, DOI: 10.1111/j.0143-2885.2004.00771.x
- Anonymous: "Apothekenbetriebsordnung: Praxisbeispiel Teil 4 - Rezepturarzneimittel", Pharmazeutische Zeitung 31/12, 1 August 2012 (2012-08-01), XP055486877, Retrieved from the Internet: URL:https://www.pharmazeutische-zeitung.de /index.php?id=42926 [retrieved on 2018-06-21]
- Anonymous: "GNPD-Mouthwash with propylene glycol overview", , 1 September 2014 (2014-09-01), XP055488032, Retrieved from the Internet: URL:www.gnpd.com [retrieved on 2018-06-26]

## Beschreibung

Die vorliegende Erfindung betrifft eine Zusammensetzung für die Mundpflege.

Die zerstörerische Wirkung von Karies auf Zahnoberflächen und Zähne insgesamt ist gemeinhin bekannt. So leiden ungefähr 90% der erwachsenen Personen an Karies. Ferner sind Krankheiten, insbesondere entzündliche Veränderungen, des Zahnfleisches (Gingiva) weit verbreitet. Aus einer Gingivitis kann sich dabei rasch eine Parodontitis entwickeln. Ein ursächlicher Zusammenhang zwischen dem Vorhandensein von Zahnbelag (Plaque) und insbesondere von Zahnstein und verschiedenen Erkrankungen der Gingiva ist dabei allgemein anerkannt.

Als Basis für eine geeignete Prophylaxe gilt allgemein das zwei- oder mehrmals am Tag durchzuführende Zähneputzen, um eine unerwünschte Belagbildung auf den Zahnoberflächen und insgesamt einen bakteriellen Befall des Mundraums zumindest einzudämmen.

Allerdings lässt sich bereits wenige Minuten nach dem Zähneputzen die Neubildung der sog. Pellikelschicht auf den Zahnoberflächen nachweisen. Die Bezeichnungen "Pellikelschicht" und "Pellikel" werden hier und im Folgenden synonym verwendet. Dabei handelt es sich um einen Niederschlag aus Proteinen aus dem Speichel. Die Pellikel ist für die Zahngesundheit an sich wenig bedenklich. Allerdings siedeln sich im Folgenden auf der Pellikel weitere Mikroorganismen aus der Speichelflora an, was zu einem Dickenwachstum des entstandenen Biofilms führt. Hierbei können sich auch schädliche Mikroorganismen ansiedeln. Dabei kann eine Matrix aus extrazellulären polymeren Substanzen (EPS) entstehen, welche verschiedenste Bakterienspezies auf der Zahnoberfläche einzubetten vermag. Die entstehende Zellschicht wird als Zahnbelag (Plaque) bezeichnet und fördert das Auftreten von Karies immens.

Aus der Plaque kann durch Aufnahme und Einlagerung anorganischer Stoffe (Mineralstoffe) aus dem Speichel Zahnstein entstehen. Dieser ist i.A. nicht mehr durch die Zahnbürste entfernbar. Der Zahnstein fördert das Auftreten der oben angeführten Erkrankungen der Gingiva.

Aus dem Stand der Technik sind Zusammensetzungen bekannt, deren Anwendung zumindest das Reinigen der Zahnoberflächen und so das Eindämmen oder die Entfernung von Plaque bewirken soll.

Aus DE 10 2008 033 105 A1 sowie DE 10 2008 039 681 A1 sind Mund- und Zahnpflege- und -reinigungsmittel bekannt, welche vorzugsweise in Form bekannter Zahnpasten ausgestaltet sind. Diese enthalten in einem fließfähigen Träger suspendierte Partikel, welche u.a. synthetische Materialien aufweisen können. Heutzutage werden vom Markt jedoch vermehrt Produkte ohne derartige Partikel gefordert. Des Weiteren ist die Anwendung dieser bekannten Reinigungsmittel üblicherweise auf das Zähneputzen beschränkt, womit allerdings die oben beschriebene Neubildung eines Biofilms nach dem Putzen regelmäßig einhergeht. Schließlich ist das Zähneputzen nicht an jedem Ort und in unauffälliger Weise möglich.

DE 200 13 336 U1 beschreibt ein ölbasiertes Mund- und Zahnpflegegel, das bei der Prophylaxe und/oder Behandlung von entzündlichen Erkrankungen des Zahnbettes und/oder des Zahnhalses Anwendung finden soll. Dazu enthält das Gel eine Zubereitung aus verschiedenen Vitaminen. Allerdings ist in Bezug auf diese Zubereitung keine Wirkung auf unerwünschte Beläge auf Zahnoberflächen dargetan.

Anonymous, "isopropyl myristate mouthwash | Mistral How To", (2012-03-05), URL: https://mistralhowto.wordpress.com/tag/isopropyl-myristate-mouthwash/, (gefunden am 2016-01-08), und:
Anonymous, "Dentyl pH - Wikipedia, the free encyclopedia", (2012-07-15), URL:https://web.archive.org/web/20120715054306/http://en.wikipedia.org/wiki/D entyl_pH, (gefunden am 2016-01-08), offenbaren Zusammensetzungen zur Mundpflege, welche Isopropylmyristat aufweisen.

S. SHAPIRO, "The inhibitory action of fatty acids on oral bacteria", ORAL MICROBIOLOGY AND IMMUNOLOGY., DK, (1996-10-01), vol. 11, no. 5, ISSN 0902-0055, Seiten 350 - 355, diskutiert allgemein die Wirksamkeit von Myristaten gegen Mundbakterien.

PARSAEE SIAMAK ET AL, "In-vitro release of diclofenac diethylammonium from lipid-based formulations", INTERNATIONAL JOURNAL OF PHARMACEUTICS, ELSEVIER BV, NL, (2002-07-08), vol. 241, no. 1, ISSN 0378-5173, Seiten 185 - 190, beschreibt die Freisetzung des nichtsteroidalen Antirheumatikums Diclofenac aus lipidbasierten Zusammensetzungen zur topischen, äußerlichen Anwendung. ABDEL-BAR HEND MOHAMED ET AL, "Evaluation of brain targeting and mucosal integrity of nasally administrated nanostructured carriers of a CNS active drug, clonazepam.", JOURNAL OF PHARMACY & PHARMACEUTICAL SCIENCES : A PUBLICATION OF THE CANADIAN SOCIETY FOR PHARMACEUTICAL SCIENCES, SOCIETE CANADIENNE DES SCIENCES PHARMACEUTIQUES 2013, (2013), vol. 16, no. 3, ISSN 1482-1826, Seiten 456 - 469, befasst sich mit der Freisetzung von Clonazepam bei der nasalen Gabe innerhalb einer Trägerzusammensetzung. Clonazepam ist ein Mittel zur Behandlung zerebraler Krampfanfälle.

WO 2009/092040 A2 offenbart schmerzstillende Zusammensetzungen zur topischen, äußeren Anwendung. Solche Formulierungen enthalten bspw. das Analgetikum Dimethylsulfoxid (DMSO), das Lokalanästhetikum Benzocain, oder auch Capsaicin.

WO 00/6113 A1 offenbart u.a. ein Diclofenac enthaltendes Schmerzgel, welches für die topische Anwendung geeignet ist.

KINNUNEN T ET AL: "ANTIBACTERIAL AND ANTIFUNGAL PROPERTIES OF PROPYLENE GLYCOL, HEXYLENE GLYCOL, AND 1,3-BUTYLENE GLYCOL IN VITRO", ACTA DERMATO-VENEREOLOGICA, TAYLOR & FRANCIS LTD, UNITED KINGDOM, Bd. 71, Nr. 2, 1. Januar 1991 (1991-01-01), ISSN: 0001-5555, Seiten 148-150, diskutiert die Vorteilhaftigkeit von Hexylenglykol gegenüber Propylenglycol für die Verwendung in kosmetischen und dermatologischen Trägersubstanzen.

T. Takushige ET AL: "Endodontic treatment of primary teeth using a combination of antibacterial drugs", INTERNATIONAL ENDODONTIC JOURNAL, Bd. 37, Nr. 2, 1. Februar 2004 (2004-02-01), ISSN: 0143-2885, Seiten 132-138, betrifft Untersuchungen an einer Mischung verschiedener antibakterieller Wirkstoffe in einer Salbe zur Behandlung von infizierten Zahnwurzelkanälen. Die Salbe weist dabei Macrogol (dabei handelt es sich um Polyethylenglykol) und Propylenglycol auf.

Anonymous: "Apothekenbetriebsordnung: Praxisbeispiel Teil 4 - Rezepturarzneimittel", Pharmazeutische Zeitung 31/12, 1. August 2012 (2012-O8-01), Gefunden im internet: URL: https://www.pharmazeutische zeitung.de/index.php?id=42926 [gefunden am 2018-06-21], und:
Anonymous: "GNPD-Mouthwash with propylene glycol overview", 1. September 2014 (2014-O9-O1), Gefunden im internet: URL: www.gnpd.com [gefunden am 2018-06-26], beschreiben weitere Zusammensetzungen mit Propylenglycol.

Der vorliegenden Erfindung liegt die Aufgabe zugrunde, zumindest einen der oben aufgeführten Nachteile zu lindern.

Des Weiteren liegt der Erfindung die Aufgabe zugrunde, einen Beitrag zur antibakteriellen Behandlung des Mundraums, insbesondere von Zahnoberflächen, zu leisten.

Schließlich liegt der Erfindung die Aufgabe zugrunde, einen Beitrag zu einer einfachen Anwendung einer Zusammensetzung für die Mundpflege zu leisten.

Einen Beitrag zur Lösung mindestens einer der vorstehend genannten Aufgaben leistet eine Zusammensetzung für die Mundpflege mit den Merkmalen des Anspruchs 1.

Vorteilhafte Ausgestaltungen und Weiterbildungen ergeben sich aus den nachgeordneten Ansprüchen.

Im Rahmen der vorliegenden Erfindung hat sich gezeigt, dass die erfindungsgemäße Zusammensetzung für die Mundpflege eine überraschend gute Wirkung bei der Inhibierung und der Reduktion von Biofilmen auf Zahnoberflächen zeigt. Die Zusammensetzung ist in überraschender Weise sehr gut dazu geeignet, die Ausbildung derartiger Biofilme auf Zahnoberflächen zu kontrollieren oder sogar gänzlich zu verhindern. Dabei wird die Pellikelbildung modifiziert. Die Neubildung genannter Biofilme wird auch nach der Anwendung nachhaltig gestört.

Die Anwendung der erfindungsgemäßen Zusammensetzung auf der gereinigten Zahnoberfläche beeinflusst und modifiziert die Pellikelbildung dergestalt, dass die Adhärenz von Bakterien nachhaltig reduziert wird.

Folglich kann bereits die Entstehung von Zahnbelag (Plaque) kontrolliert oder sogar unterbunden werden. Schädliche Bakterien können sich nicht mehr an den Zahnoberflächen anlagern.

Zusätzlich zeigt sich eine bemerkenswerte antibakterielle Wirkung der erfindungsgemäßen Zusammensetzung für die Mundpflege.

Die erfindungsgemäße Zusammensetzung kann als homogenes (einphasiges) Spray oder Gel im Mundraum angewendet werden. Dadurch wird die Anwendung vereinfacht. Eine Zahnbürste ist dabei zum Auftragen der Zusammensetzung nicht zwingend erforderlich, kann aber nach Wunsch unterstützend benutzt werden. Eine Anwendung als Mundspülung, Paste oder dgl. ist alternativ möglich.

Darüber hinaus lässt sich die erfindungsgemäße Zusammensetzung vergleichsweise kostengünstig herstellen.

Die erfindungsgemäße Zusammensetzung erweist sich als besonders vorteilhaft, sofern sie als Zubereitung zum Sprühen oder als Spray oder als Mundspüllösung ausgebildet ist.

In einer Alternative erweist sich die Ausgestaltung als Lutschtablette, Pastille, Bonbon oder Kaugummi ebenfalls als vorteilhaft. Eine weitere besondere Ausgestaltung betrifft einen Haftstreifen zum Aufbringen auf die Zähne. Solche Haftstreifen sind, mit einem geeigneten Wirkstoff versehen, für sich gesehen bekannt, um die Aufhellung bzw. das Bleichen von Zähnen zu bewirken. Im Rahmen der vorliegenden Erfindung wird nun vorgeschlagen, die erfindungsgemäße Zusammensetzung mittels eines solchen Haftstreifens in möglichst dauerhaften Kontakt mit den Zahnoberflächen zu bringen.

Alternativ, aber ebenfalls bevorzugt, kann die Zusammensetzung als Gel oder gelartig ausgebildet ist, insbesondere wobei die Zusammensetzung zumindest ein Geliermittel, vorzugsweise Polyacrylsäure und/oder Xanthan und/oder Triethanolamin, aufweist.

Im Rahmen der Erfindung wird die Verwendung der erfindungsgemäßen Zusammensetzung zur Mund- und/oder Zahnpflege und/oder zur oralen Prophylaxe vorgeschlagen.

### Beispiele

Die erfindungsgemäße Zusammensetzung ist im Rahmen des nachfolgend aufgeführten Beispiels 1 angegeben.

Sämtliche Zahlenwerte verstehen sich dabei als Massen-%, bezogen auf die gesamte Masse der Zusammensetzung.

### Beispiel 1

| | |
|---|---|
| Isopropylmyristat | 67,5 |
| Propylenglycol | 20,0 |
| Lecithin | 12,5 |

### Versuche

Im Folgenden werden Versuchsergebnisse erörtert, welche mit der erfindungsgemäßen Zusammensetzung gemäß **Beispiel 1** aus dem vorhergehenden Abschnitt "Beispiele" erhalten worden sind.

Grundlage der Versuchsreihen waren sind dabei einheitlich hergestellte Prüfkörper aus bovinem Zahnschmelz (Zahnschmelzprüfkörper) gewesen, welche von Versuchspersonen über einen Zeitraum von 24h in der Mundhöhle (in vivo) getragen worden sind.

Die **Figuren 1** **und** **2** zeigen elektronenmikroskopisch erhaltene Aufsicht- bzw. Schnittbilder dieser Zahnschmelzprüfkörper, welche von zwei unterschiedlichen Versuchspersonen (Versuchsperson 1: Fig. 1, Versuchsperson 2: Fig. 2) in der Mundhöhle getragen worden sind.

Während der 24-stündigen Exposition der Schmelzprüfkörper in der Mundhöhle hat jede der Versuchspersonen im Abstand von 12h insgesamt zweimal für jeweils 30 Sekunden ihren Mund mit der Zusammensetzung gemäß **Beispiel 1** gespült. Alle sonstigen Mundhygienemaßnahmen sind hingegen unterblieben.

**Figur 3** hingegen zeigt als Kontrolle ein Aufsichtsaufnahme auf einen wie oben erläuterten Prüfkörper, der von einer Versuchsperson 24h in vivo getragen worden ist, wobei hier weder ein Spülen mit einer erfindungsgemäßen Zusammensetzung noch irgendwelche sonstigen Mundhygienemaßnahmen durchgeführt worden sind.

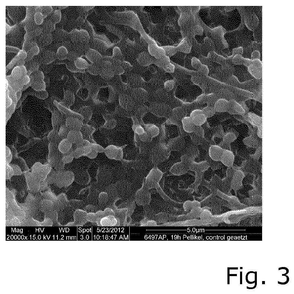

Fig. 3 belegt, dass sich innerhalb von 24h ohne zwischenzeitliche Mundhygiene auf der Zahnschmelzoberfläche unter Mundhöhlenbedingungen ein dichter Biofilm ("Bakterienrasen") ausbildet. In der rasterelektronenmikroskopischen Aufsicht imponieren bei höherer Vergrößerung kugelförmige Bakterien, die als mehrlagiger Biofilm die Schmelzoberfläche bedecken und vollständig maskieren.

Wird während der 24-stündigen Exposition der Schmelzprüfkörper in der Mundhöhle im Abstand von 12h zweimal für lediglich jeweils 30s mit der Lösung gemäß **Beispiel 1** gespült, so ist die Biofilmneubildung im Vergleich zu dem Kontrollergebnis aus Fig. 3 allerdings deutlich reduziert.

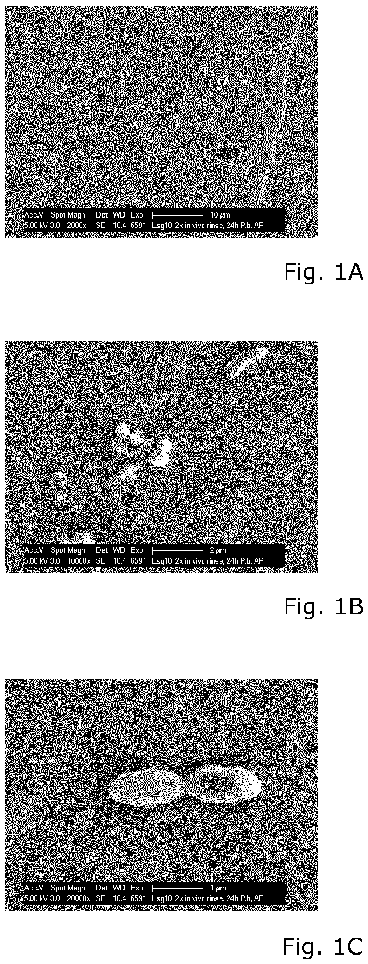

Wie in **Fig. 1** gut zu sehen ist, lassen sich dann in verschiedenen Vergrößerungen nur noch einzelne Inseln von adhärenten Bakterien, jedoch kein geschlossener Biofilm wie bei Fig. 3, in der rasterelektronenmikroskopischen Aufsicht auf die Schmelzoberfläche darstellen.

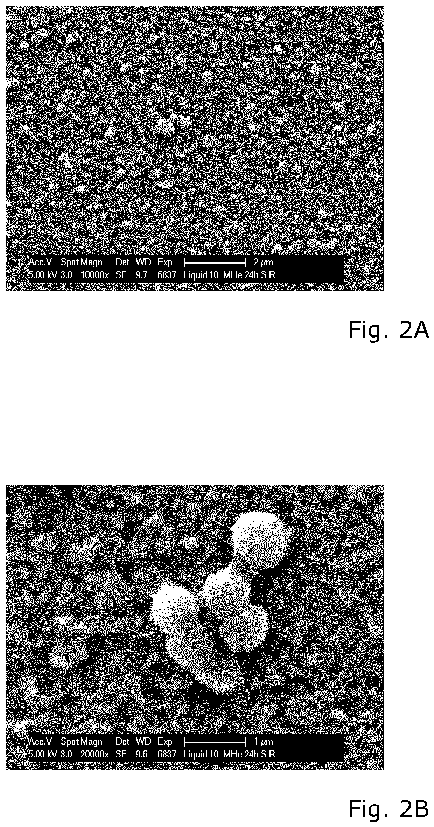

Dieses überraschend günstige Ergebnis konnte bei einer zweiten Versuchsperson vollumfänglich bestätigt werden, vgl. **Fig. 2****.**

Die Zahnschmelzoberfläche ist bei Verwendung der erfindungsgemäßen Zusammensetzung von einer granulär erscheinenden Proteinschicht (Pellikelschicht) bedeckt, vgl. Fig. 1B und auch Fig. 2A. Diese Pellikel trägt zur partiellen Maskierung der Zahnschmelzoberfläche bei.

Auf der entstandenen Pellikelschicht sind in hoher Vergrößerung lediglich einzelne Bakterienansammlungen identifizierbar, vgl. Fig. 1C und Fig. 2B. Es entsteht jedoch gerade kein dichter Biofilm, wie dies bei Fig. 3 der Fall ist.

Fig. 1 und 2 zeigen im Ergebnis, dass nach lediglich zweimaligem Spülen mit einer Lösung mit der Zusammensetzung von Beispiel 1 über jeweils 30s im Abstand von 12h die 24h-Biofilmbildung unter Mundhöhlenbedingungen nahezu vollständig unterbunden (inhibiert) werden konnte. Nach 24-stündiger intraoraler Exposition ist die Schmelzoberfläche unter dem Einfluss der zweimaligen Spülung mit der Lösung nach Beispiel 1 von einer Pellikelschicht, die sich im rasterelektronenmikroskopischen Bild netzartig granulär darstellt, partiell maskiert. Auf der Pellikel lassen sich nur (sehr vereinzelt) einzelne adhärente Bakterien detektieren.

## Patentansprüche

1. Zusammensetzung für die Mundpflege, wobei die Zusammensetzung aufweist (in Massen-%):
| | |
|---|---|
| Isopropylmyristat: | 67,5% |
| Propylenglycol: | 20% |
| Lecithin: | 12,5% |

2. Zusammensetzung nach Anspruch 1, **dadurch gekennzeichnet, dass** die Zusammensetzung als Zubereitung zum Sprühen oder als Spray oder als Mundspülzusammensetzung ausgebildet ist.

3. Zusammensetzung nach Anspruch 1 oder 2, **dadurch gekennzeichnet, dass** die Zusammensetzung als Gel oder gelartig ausgebildet ist.

4. Zusammensetzung nach einem der Ansprüche 1 bis 3 zur Verwendung zur Mund- und/oder Zahnpflege und/oder zur oralen Prophylaxe.

5. Zusammensetzung nach einem der Ansprüche 1 bis 3 zur Anwendung in einem Verfahren zur Mund- und/oder Zahnpflege und/oder zur oralen Prophylaxe, wobei das Verfahren die Anwendung der Zusammensetzung im Mundraum, und insbesondere auf Zahnoberflächen, aufweist.

## Claims

1. A composition for oral care, the composition comprising (based on mass %):
isopropyl myristate: 67.5%
propylene glycol: 20%
lecithin: 12,5%

2. The composition according to claim 1, **characterized in that** the composition is formed as a preparation for spraying or as a spray or as a mouthwash composition.

3. The composition according to claim 1 or 2, **characterized in that** the composition is in the form of a gel or in gelatinous form.

4. The composition according to one of claims 1 to 3 for use in oral and/or dental care and/or in oral prophylaxis.

5. The composition according to one of claims 1 to 3 for use in a process for oral and/or dental care and/or for oral prophylaxis, the process comprising application of the composition within the oral cavity, and in particular on tooth surfaces.

## Revendications

1. Composition pour l'hygiène buccale, la composition comprenant (en % en masse):
myristate d'isopropyle : 67,5%
propylène glycol ; 20%
lécithine : 12,5%

2. Composition selon la revendication 1, **caractérisée en ce que** la composition se présente sous la forme d'une préparation en spray ou sous la forme d'un spray ou d'un bain de bouche.

3. Composition selon la revendication 1 ou 2, **caractérisée en ce que** la composition se présente sous forme d'un gel ou sous forme gélatineuse.

4. Composition selon l'une des revendications 1 à 3 pour l'utilisation dans le cadre de soins bucco-dentaires et/ou de prophylaxie orale.

5. Composition selon l'une des revendications 1 à 3, destinée à être utilisée dans un procédé de soins bucco-dentaires et/ou de prophylaxie bucco-dentaire, le procédé comprenant l'application de la composition dans la cavité buccale, et en particulier sur la surface des dents.
